# EUROPÄISCHE PATENTSCHRIFT

(11) **EP 0 308 865 B1**
(45) Veröffentlichungstag und Bekanntmachung des Hinweises auf die Patenterteilung: **03.02.1993**
(21) Anmeldenummer: 88115393.6
(22) Anmeldetag: 20.09.1988
(51) Int. Cl.: C07C 57/04, C07C 51/377

(54) **Verfahren zur Oxydehydrierung von Isobuttersäure**
Process for the oxydehydrogenation of isobutyric acid
Procédé pour l'oxydéshydrogénation d'acide isobutyrique

(30) Priorität: 24.09.1987 DE 3732106
(43) Veröffentlichungstag der Anmeldung: 29.03.1989
(73) Patentinhaber: Röhm GmbH, 64293 Darmstadt (DE)
(72) Erfinder: Ruppert, Wolfgang, Dr. Ing., D-6104 Seeheim-Jugenheim (DE); Siegert, Hermann-Josef, Dr., D-6104 Seeheim-Jugenheim (DE)

(56) Entgegenhaltungen:
- EP-A- 0 046 840
- EP-A- 0 255 640
- DE-A- 3 145 091

## Beschreibung

Die Erfindung betrifft ein kontinuierliches Verfahren zur Oxydehydrierung von Isobuttersäure zu Methacrylsäure durch Umsetzung mit Sauerstoff in der Gasphase an einem heterogenen Katalysator.

Die Oxydehydrierung von Isobuttersäure zu Methacrylsäure ist zwar in der Patentliteratur mehrfach beschrieben, wird aber noch nicht kontinuierlich im technischen Maßstab durchgeführt. Voraussetzung für die technische Verwirklichung des Verfahrens sind eine hohe Ausbeute und Selektivität und die Möglichkeit der ununterbrochenen Betriebsführung über lange Zeiträume.

Nach DE-A 3 248 600 und 3 145 091 erreicht man einen Umsatz der eingesetzten Isobuttersäure bis zu 86 % und eine Selektivität für Methacrylsäure bis zu 82 %, wenn man einen vanadin- und kupferhaltigen Katalysator auf Basis einer Phosphor-Molybdän-Heteropolysäure auf einem Trägermaterial von geringer spezifischer Oberfläche einsetzt. Als Reaktoren werden herkömmliche Rohrreaktoren oder Kreislaufreaktoren oder Kombinationen dieser Reaktortypen vorgeschlagen.

Der Einsatz eines Kreislaufreaktors für Oxydehydrierungsverfahren in der Gasphase durch heterogene Katalyse wurde erstmals in der DE-A 3 019 731 vorgeschlagen. Dabei erwies es sich als vorteilhaft, daß man auf den sonst bei Oxydehydrierungverfahren üblichen Einsatz großer Mengen an Inertgasen, wie Wasserdampf oder Stickstoff, teilweise oder völlig verzichten konnte. Wasserdampf wurde bisher als unentbehrlich für die Aufrechthaltung der Katalysatoraktivität über lange Zeit angesehen. Der Verzicht auf Wasserdampf und andere Inertgase vereinfacht die Weiterverarbeitung des Oxydehydrierungsproduktes erheblich.

Beim Arbeiten im Kreislaufreaktor wird der Verzicht auf verdünnende Inertgase dadurch ermöglicht, daß das frisch zugeführte Reaktionsgas vor Eintritt in den Reaktorraum mit dem rückgefuhrten Kreisgas verdünnt wird. Das Kreisgas enthalt als Nebenprodukt der Oxydehydrierungsreaktion Wasserdampf, so daß sich der Zusatz von weiterem Wasserdampf mit dem Frischgas teilweise oder ganz erübrigt. Kreislaufreaktoren erfordern allerdings einen beträchtlichen Energieaufwand zur Umwälzung großer Gasmengen. Ein weiterer Nachteil liegt in der begrenzten Betriebsdauer einer Katalysatorfüllung und in der häufigen Betriebsunterbrechung zum Ersatz des Katalysators.

In der US-A 4 232 174 wird die Oxydehydrierung von Isobuttersäure an einem Eisenphosphat-Katalysator in einem Wirbelbettreaktor beschrieben. Das Reaktionsgas wird mit etwa 4 bis 10 Mol Wasserdampf je Mol Isobuttersäure verdünnt. Der Umsatz der Isobuttersäure liegt mit maximal 63 % unter dem für ein technisches Verfahren erforderlichen Niveau.

### Aufgabe und Lösung

Es war das Ziel der Erfindung, das kontinuierliche Verfahren über lange, von der Standzeit des Katalysators unabhängige Zeiträume ununterbrochen auf einem hohen Niveau des Umsatzes und der Selektivität durchzuführen. Die Standzeit, also die Zeitspanne, in der die katalytische Aktivität des verwendeten Katalysatormaterials nicht auf unwirtschaftliche Werte abfällt, beträgt bei den zur Oyxdehydrierung von Isobuttersäure einsetzbaren Katalysatoren durchweg nur wenige Monate. Betriebsunterbrechungen zum Austausch des Katalysators in so kurzen Abständen wären wirtschaftlich nicht tragbar.

Es wurde gefunden, daß die Inaktivierung des Kataysators mit einer Mo-V-P-Heteropolysäure als aktiver Substanz während einer langen kontinuierlichen Betriebsdauer beim Einsatz von weniger als 2 Mol Wasserdampf pro Mol Isobuttersäure im Wirbelbettreaktor langsamer vonstatten geht, als in einem Rohrreaktor mit der gleichen Katalysatormenge unter sonst gleichen Betriebsbedingungen. Dadurch werden die Voraussetzungen für eine lange ununterbrochene Betriebsdauer entscheidend verbessert.

Das Ziel einer praktisch unbegrenzten, jedenfalls nicht von der Katalysatorstandzeit abhängigen Betriebsdauer wird erfindungsgemäß dadurch erreicht, daß bei der Umsetzung am heterogenen Katalysator im Wirbelbett ein Teil des Katalysators während des kontinuierlichen Betriebs durch frisches Katalysatormaterial ersetzt wird. Darunter wird neu erzeugtes oder regeneriertes Katalysatormaterial von hoher Aktivität verstanden. Der Wirbelbett-Katalysator hat vor Festbettreaktoren, wie sie bei Rohrreaktoren oder bei Kreislaufreaktoren vorliegen, den Vorteil, daß während des laufenden Betriebs ein kontinuierlicher oder anteilsweiser Ersatz des Katalysators möglich ist.

Es muß als überraschend angesehen werden, daß es sich als möglich erwies, bei der Anwendung des Wirbelbettreaktors und bei Einsatz von Sauerstoff in Form von Luft auf den Zusatz von Inertgasen wenigstens insoweit zu verzichten, als keine über den Luftstickstoff, der zusammen mit dem zur Oxydehydrierung benötigten Sauerstoff eingeführt wird, hinausgehenden Inertgasmengen eingesetzt zu werden brauchen. Vor allem war nicht vorauszusehen, daß beim Verfahren der Erfindung auf den Zusatz von Wasserdampf weitgehend verzichtet werden kann, denn gemäß DE-A 3 019 731 kann nur in einem Kreislaufreaktor wegen der dort eintretenden Verdünnung des Reaktionsgases mit dem wasserdampfhaltigen Kreislaufgas auf den Inertgaszusatz verzichtet werden. Zwar wirkt sich auch beim Verfahren der Erfindung jede Verdünnung des Reaktionsgases vorteilhaft auf die Selektivität aus. Um aber die bei der Verdünnung mit Inertgasen auftretenden Nachteile, vor allem bei der Gewinnung der gebildeten Methacrylsäure, auszuschließen, wird erfindungsgemäß nur die Verdünnung mit rückgeführtem Abgas, aus dem die kondensierbaren Bestandteile bereits entfernt sind, und der Betrieb bei unteratmosphärischem Druck in Betracht gezogen. Beide Maßnahmen führen zur Verminderung der Partialdrücke an Isobuttersäure-Dampf und Sauerstoff, ohne das Reaktionsgas mit Fremdgasen zu vermischen.

Im gleichen Sinne wirkt sich die getrennte Zugabe von Sauerstoff und Isobuttersäure an zwei oder mehr Stellen in das Wirbelbett aus. Es wurde festgestellt, daß sich der Sauerstoff in Abwesenheit von Isobuttersäure mit dem Katalysator umzusetzen vermag und daß der auf diese Weise aufgeladene Katalysator den Sauerstoff bei einer nachfolgenden Oxydehydrierungsreaktion abgibt. Der Schritt der Sauerstoffbeladung das Katalysators läßt sich daher verfahrensmäßig wenigstens teilweise von der Umsetzung der Isobuttersäure trennen. Dazu können verschiedene Zonen des gleichen Wirbelbettes oder getrennte Wirbelbetten dienen.

### Ausführung der Erfindung

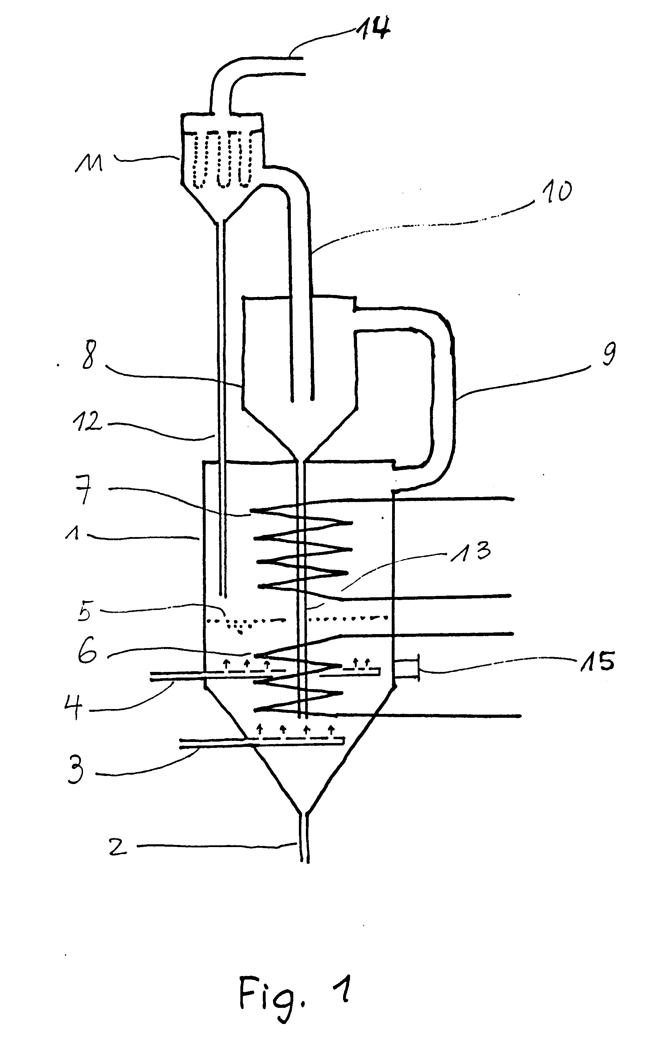

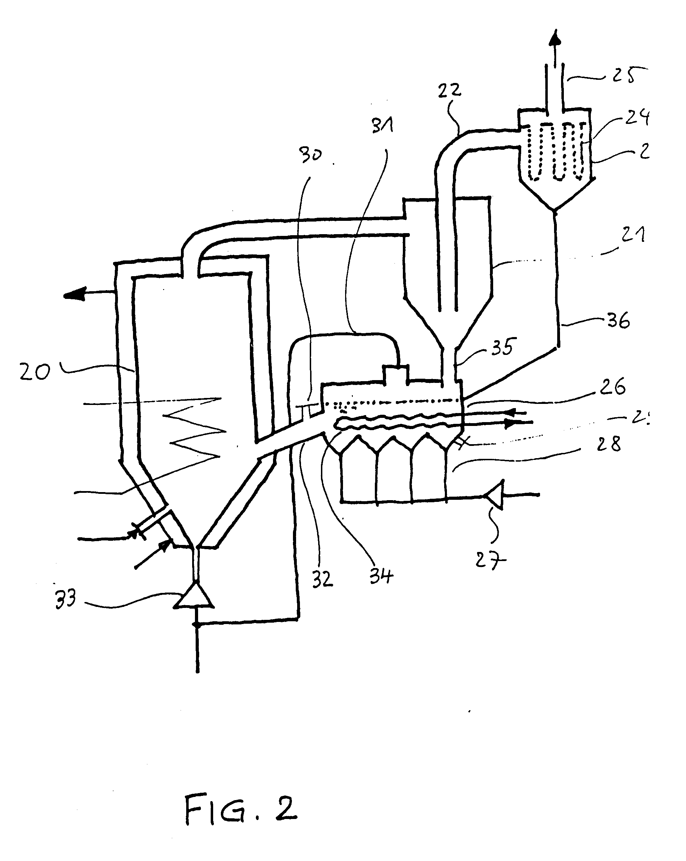

Vorteilhafte Ausführungsformen des Verfahrens der Erfindung werden nachfolgend anhand der Figuren 1 und 2 näher beschrieben.
**Figur 1** zeigt schematisch einen vertikalen Schnitt durch einen einstufigen Wirbelbettreaktor.
**Figur 2** zeigt in gleicher Darstellungsweise einen zweistufig aufgebauten Wirbelbettreaktor.

In den einstufigen Wirbelbettreaktor 1 gemäß Fig.1 wird durch die Bodenleitung 2 entweder das gesamte Reaktionsgas oder vorzugsweise nur Sauerstoff bzw. Luft eingeführt. In diesem Falle wird dampfförmige oder flüssige Isobuttersäure durch eine oder mehrere Leitungen 3, 4 in Strömungsrichtung hinter der Einleitungsstelle des Sauerstoffes in das Wirbelbett eingeführt. Flüssige Isobuttersäure wird durch den Wärmeinhalt des wirbelnden Katalysators rasch verdampft.

Für die Oxydehydrierung von Isobuttersäure ist eine große Zahl von heterogen in der Gasphase wirkenden Katalysatoren beschrieben. Für das Verfahren der Erfindung wird als Katalysator eine Phosphor-Molybdän-Heteropolysäure, die mit weiteren Metallen, insbesondere Vanadin und Kupfer, modifiziert sein kann, eingesetzt, wobei solche auf einem Trägermaterial mit einer spezifischen Oberfläche von weniger als 2 qm/g besonders bevorzugt sind. Für die Ausbildung des Wirbelbettes ist eine Korngröße von 30 bis 300 Mikrometer geeignet.

Bei einer Strömungsgeschwindigkeit der Gasphase von 5 bis 50 cm/sec bildet sich ein Wirbelbett mit einer Dichte zwischen etwa 100 und 2000 kg/cbm aus. In dem in Fig.1 dargestellten Reaktortyp bildet sich in der Regel eine ziemlich scharfe Obergrenze 5, die etwa in der Mitte des Reaktors liegen sollte. In Reaktoren von dem in Fig.2 dargestellten Typ gibt es keine derartige Grenze und die Dichte des Wirbelbettes ist geringer, z.B. 100 bis 1000 kg/cbm.

Die Oxydehydrierung verläuft exotherm und wird durch Kühlung bei einer gleichbleibenden Reaktionstemperatur von 300 bis 400°c, vorzugsweise 320 bis 380°C, gehalten. Es ist zweckmäßig, einen Teil der Reaktionswärme mittels eines Wärmetauschers 6 innerhalb des Wirbelbettes und einen weiteren Teil mit dem Wärmetauscher 7 oberhalb des Wirbelbettes zu entziehen. Weiterhin kann die Reaktorwandung gekühlt werden. Die Wirtschaftlichkeit des Verfahrens kann gesteigert werden, wenn die Reaktionswärme zur Dampferzeugung genutzt wird.

Das aus dem Wirbelbettreaktor 1 abströmende Reaktionsgas tritt über die Leitung 9 in einen Zyklonabscheider 8 und von dort über eine Leitung 10 in die Filterkammer 11 ein. In beiden Geräten wird mitgerissener Katalysatorstaub vom Reaktionsgas getrennt und durch Fallrohre 12, 13 in den Wirbelschichtreaktor zurückgeleitet. Das gereinigte Reaktionsgas wird über die Leitung 14 abgenommen und in die Aufarbeitungsanlage eingeleitet. Ein Teil des Katalysators kann über Stutzen 15 während des Betriebs abgenommen und nach Aufarbeitung und Regenerierung zurückgeführt werden.

In dem zweistufigen Wirbelbettreaktor gemäß Figur 2 wird der Oxydehydrierungsreaktor 20 so betrieben, daß er von dem Wirbelbett ganz ausgefüllt wird und laufend ein Teil des Katalysators in den Zyklonabscheider 21 hinüber gefördert wird. Das Reaktionsgas strömt über die Leitung 22 weiter in die Filterkammer 23 und wird nach Durchtritt durch die Filterkerzen 24 über die Leitung 25 abgenommen. Der aus dem Gasstrom abgeschiedene Katalysator gelangt uber Leitungen 35, 36 in den Regenerierungs-Wirbelbettreaktor 26. Dort wird mittels eines Gebläses über Leitungen 28 Sauerstoff oder Luft in das Wirbelbett eingeleitet. Dabei werden Abscheidungen von verkohltem organischem Material von der Oberfläche des Katalysators abgebrannt, der Katalysator regeneriert und mit Sauerstoff aufgeladen. Die freiwerdende Verbrennungswärme wird mittels eines Wärmetauschers 34 entzogen, um eine für die Aktivität schädliche Überhitzung des Katalysators zu vermeiden, und zweckmäßigerweise zur Dampferzeugung genutzt. Da eine vollständige Regenerierung des Katalysators im Reaktor 26 nicht immer erreicht wird, wird ein Teil des Katalysators über Stutzen 29, 30 während des Betriebs entnommen und nach Aufarbeitung und Regenerierung zurückgeführt. Der regenerierte Katalysator fließt aus dem Reaktor 26 über das Fallrohr 32 in den Oxydehydrierungsreaktor 20 zurück.

Das aus dem Reaktor 26 über die Leitung 31 abströmende Gas kann gegebenenfalls einer Abgasentsorgung zugeführt werden, enthalt aber in der Regel noch soviel Dehydrierungsprodukt, daß es in den Reaktionsgasstrom eingeleitet werden kann, beispielsweise über das Gebläse 33 in die in den Oxydehydrierungsreaktor einzuleitende Luft oder in den oberen Bereich des Reaktors 20 oder den Zyklonabscheider 21.

Die Austauschrate des Katalysatormaterials muß von dessen Standzeit sowie von dem wirtschaftlich vertretbaren Verlust an Katalysatoraktivität abhängig gemacht werden. In der Regel wird die gesamte Katalysatorfüllung in einem Zeitraum zwischen 1000 und 10 000 Stunden einmal ausgetauscht. Ob dies kontinuierlich oder in Anteilen, beispielsweise alle 2 bis 24 Stunden, erfolgt, ist vor allem eine Frage der Betriebsorganisation.

In dem Wirbelbett des Reaktors 1 bzw. 20 wird dampfförmige Isobuttersäure durch heterogene Katalyse mit Sauerstoff in Methacrylsäure übergeführt. Neben dieser Hauptreaktion treten in begrenztem Umfang Nebenreaktionen auf, die zu Essigsäure, Aceton und anderen, z.T. hochsiedenden Nebenprodukten führen. Sauerstoff wird beim Verfahren der Erfindung vorzugsweise in überstöchiometrischer Menge, bez. auf Isobuttersäure, eingesetzt; z.B. 1,3 bis 1,8 Mol Sauerstoff pro Mol Isobuttersäure. Die Nebenreaktionen werden umso mehr unterdrückt, je niedriger die Partialdrücke von Isobuttersäure und Sauerstoff liegen. Die Verminderung der Partialdrücke durch Zumischen von Inertgasen ist nachteilig, weil sie die Gewinnung konzentrierter Methacrylsäure aus dem Reaktionsgas erschweren.

Der völlige Verzicht auf Inertgas ist jedoch unzweckmäßig, weil vorzugsweise Sauerstoff in Form von Luft eingesetzt wird. Dadurch wird die etwa vierfache Volumenmenge an Stickstoff eingeführt. Diese Inertgasmenge erweist sich beim Verfahren der Erfindung als ausreichend, so daß auf zusätzlichen Stickstoff oder Wasserdampf als Inertgas völlig verzichtet werden kann, sofern nicht zur Vermeidung des Explosionsbereichs ein gewisser Zusatz an nicht brennbaren Gasen zweckmäßig ist. Die Vorteile der Erfindung werden noch erreicht, solange weniger als 2 Mol, insbesondere weniger als 1 Mol Wasserdampf pro Mol Isobuttersäure zugesetzt werden. Im Laufe der Umsetzung entsteht Wasserdampf aus der Hauptreaktion und zum Teil aus den Nebenreaktionen. Bei der Kondensation des Reaktionsgases wird bei Einhaltung dieser Grenzen Methacrylsäure mit einem Wassergehalt von 15 bis 45 Gew.-% gewonnen, die ohne weitere Aufarbeitungsschritte der Veresterung zugeführt werden kann.

Der weitgehende Verzicht auf Inertgase führt zunächst zu hohen Partialdrücken an Isobuttersäure und Sauerstoff. Eine wirksame Methode zur Verminderung dieser Partialdrücke ohne Verdünnung des Reaktionsgases liegt in der Verminderung des Druckes, d.h. in der Durchführung der Umsetzung bei unteratmosphärischem Druck. Es wurde gefunden, daß die Selektivität des Prozesses um etwa ein Prozent je 100 mbar Unterdruck zunimmt. Es ist vorteilhaft, die Umsetzung bei einem Gesamtdruck von hochstens 1000 mbar, vorzugsweise 250 bis 700 mbar, gemessen oberhalb des Wirbelbettes, durchzuführen. Innerhalb der Wirbelschicht nimmt der Druck von oben nach unten um etwa 100mbar/m zu. Bei Drücken unter 200 mbar beginnt das Verfahren unwirtschaftlich zu werden.

Eine weitere Möglichkeit, die Partialdrücke zu senken, besteht in der Rückführung eines Teils des umgesetzten Reaktionsgases, gegebenenfalls nach Kondensation oder Teilkondensation von Methacrylsäure und Wasser, in das frisch zugeführte Reaktionsgas. Das rückgeführte Gas enthält hohe Anteile an Kohlendioxid, Kohlenmonoxid und gegebenenfalls Stickstoff. Dieses Vorgehen ist vor allem dann zweckmäßig, wenn zur Oxydehydrierung reiner Sauerstoff oder mit Sauerstoff angereicherte Luft verwendet wird, kann aber auch mit dem Einsatz von Luft und gegebenenfalls Unterdruckbetriebsweise kombiniert werden. Obwohl die Kondensation der Methacrylsäure bei dieser Verfahrensweise erschwert wird, werden die Nachteile der Arbeitsweise mit Wasserdampf als Inertgas - nämlich die Erzeugung einer stark verdünnten wäßrigen Methacrylsäure und deren Isolierung durch Extraktion - vermieden. Wird das Volumenverhältnis von Frischgas zu rückgeführtem Gas auf Werte von 1 : 0,5 bis 1 : 5 eingestellt, wird auch die Bildung eines explosiven Reaktionsgases ausgeschlossen.

Der Verminderung des Sauerstoff-Partialdruckes dient auch die Zuführung der Isobuttersäure an einer oder mehreren aufeinderfolgenden Stellen in Strömungsrichtung hinter der Zuführungsstelle des Sauerstoffes, weil dieser dann an den Katalysator gebunden wird, bevor er mit Isobuttersäuredampf in Berührung tritt. Weiterhin wird durch diese Maßnahme der Explosionsbereich sicherer umgangen.

Durch Kombination der erwähnten Maßnahmen gelingt es, den Umsatz der eingesetzten Isobuttersäure auf 90 bis 95 % bei einer Selektivität für Methacrylsäure von 75 bis 80 % zu steigern, so daß Methacrylsäure in einer Ausbeute von 70 bis 75 % gewonnen werden kann. Diese Werte erhalten noch dadurch besonderes Gewicht, daß die durch Kondensation erhaltene Methacrylsäure nicht mehr als 15 bis 45 Gew.-% Wasser enthält und in dieser Form unmittelbar zur Veresterung eingesetzt werden kann. Man kommt dabei mit einem geringen Alkoholüberschuß aus, z.B. werden pro Mol Säure 0,01 bis 1 Mol Methanol eingesetzt. Man erhält bei der Destillation des Veresterungsgemisches ein Azeotrop, aus dem sich der Wasseranteil durch Schichtentrennung abscheiden läßt. Wurde dagegen gemäß dem Stand der Technik eine stark verdünnte wäßrige Methacrylsäure gewonnen, so müßte sie entweder durch Extraktion isoliert, durch Azeotropdestillation getrocknet oder mit einem großen Alkoholüberschuß verestert werden. Im letztgenannten Fall ist die Aufarbeitung des Veresterungsgemisches wesentlich aufwendiger.

Die Vorzüge des Verfahrens der Erfindung werden durch das nachfolgend beschriebene Ausführungsbeispiel weiter verdeutlicht.

### BEISPIEL 1

In einem Wirbelbettreaktor mit einer lichten Weite von 300 mm werden 48,8 kg eines fluidisierbaren Katalysatormaterials zu einer Betthöhe von etwa 500 mm über dem Anströmboden aufgewirbelt. Das aktive Katalysatormaterial besteht aus 11 kg einer Heteropolysäure (HPS) der Zusammensetzung H_{3,6} Cu_{0,2}P Mo₁₁ V O₄₀ und ist auf einem Aluminiumoxid-Träger mit einer inneren Oberfläche von 0,5 qm/g angeordnet. Nach dem Aufheizen des Wirbelbettes mittels Heißluft auf 350 Grad C wird dampfförmige Isobuttersäure (IBA) zusammen mit Luft bei 135 Grad C in einem Molverhältnis IBA : O₂ = 1 : 1,7 durch den Anströmboden eingeleitet. Die Verweilzeit beträgt W/F = 0,8 g HPS /(g IBA/h). Die Betriebstemperatur beträgt 355 Grad C. Oberhalb des Wirbelbettes herrscht ein Druck von 0,55 bar, der durch Absaugen des Abgases mittels einer Vakuumpumpe hinter der Kondensationsstufe aufrechterhalten wird.

Nach einem anfänglichen geringfügigen Absinken der Katalysatorleistung, die auf mechanischen Abrieb von aktiver Katalysatormasse von der Teilchenoberfläche zurückgeführt wird, stellt sich ein Umsatz von 94 % der eingesetzten IBA bei einer Selektivität von 75 % für Methacrylsäure ein. Innerhalb von 1000 Betriebsstunden sinkt der Umsatz auf 88 - 90 % und nach 2000 Stunden auf 80 bis 83 %.

Bei kontinuierlichem Austausch von 1 % des Katalysatormaterials je 24 Stunden durch frisches Katalysatormaterial stellt sich ein Dauerumsatz von 88 bis 91 % ein.

### BEISPIEL 2

In einem Wirbelbettreaktor mit einer lichten Weite von 300 mm werden 85,5 kg eines fluidisierbaren Katalysatormaterials zu einer Betthöhe von etwa 500 mm über dem Anströmboden aufgewirbelt. Das aktive Katalysatormaterial besteht aus 14 kg einer Heteropolysäure (HPS) der Zusammensetzung H_{3,6} Cu_{0,2}P Mo₁₁ V O₄₀ und ist auf einem Aluminiumoxid-Träger mit einer inneren Oberfläche von 0,5 qm/g angeordnet. Nach dem Aufheizen des Wirbelbettes mittels Heißluft auf 350 Grad C wird dampfförmige Isobuttersäure (IBA) zusammen mit Luft und Wasserdampf bei 135 Grad C in einem Molverhältnis IBA : H₂O : O₂ = 1 : 1 : 1,5 durch den Anströmboden eingeleitet. Die Verweilzeit beträgt W/F = 0,8 g HPS /(g IBA/h). Die Betriebstemperatur beträgt 360 Grad C. Oberhalb des Wirbelbettes herrscht ein Druck von 0,65 bar, der durch Absaugen des Abgases mittels einer Vakuumpumpe hinter der Kondensationsstufe aufrechterhalten wird.

Nach einem anfänglichen geringfügigen Absinken der Katalysatorleistung, die auf mechanischen Abrieb von aktiver Katalysatormasse von der Teilchenoberfläche zurückgeführt wird, stellt sich ein Umsatz von 90 % der eingesetzten IBA bei einer Selektivität von 71 % für Methacrylsäure ein. Innerhalb von 1500 Betriebsstunden sinkt der Umsatz auf etwa 86 % und nach 2000 Stunden auf 80 bis 83 %. Während dieser Betriebszeit wurden 4 kg des Katalysators zum Ausgleich von Abriebverlusten zugeführt.

## Patentansprüche

1. Kontinuierliches Verfahren zur Dehydrierung von Isobuttersäure zu Methacrylsäure durch Umsetzung mit Sauerstoff in der Gasphase an einem heterogenen Katalysator im Wirbelbett, dadurch gekennzeichnet, daß ein Katalysator mit einer Mo-V-P-Heteropolysäure als aktiver Substanz verwendet wird und je Mol Isobuttersäure weniger als 2 Mol Wasserdampf eingesetzt werden.

2. Verfahren nach Anspruch 1, dadurch gekennzeichnet, daß während des kontinuierlichen Betriebs frisches Katalysatormaterial zugesetzt wird.

3. Verfahren nach Anspruch 2, dadurch gekennzeichnet, daß kontinuierlich ein Teil des Katalysators ersetzt wird.

4. Verfahren nach Anspruch 2, dadurch gekennzeichnet, daß als frisches Katalysatormaterial ein durch Regenerieren von gebrauchtem Katalysator gewonnenes Katalysatormaterial verwendet wird.

5. Verfahren nach Anspruch 4, dadurch gekennzeichnet, daß der aus dem Wirbelbett entnommene Katalysator in einem Regenerierungs-Wirbelbett mit Sauerstoff behandelt und anschließend in das erste Wirbelbett zurückgeleitet wird.

6. Verfahren nach Anspruch 5, dadurch gekennzeichnet, daß die aus dem Regenerierungs-Wirbelbett abströmende Gasphase in das erste Wirbelbett eingeleitet wird.

7. Verfahren nach den Ansprüchen 1 bis 6, dadurch gekennzeichnet, daß die Umsetzung bei unteratmosphärischem Druck durchgeführt wird.

8. Verfahren nach Anspruch 7, dadurch gekennzeichnet, daß es bei einem Druck von höchstens 700 mbar durchgeführt wird.

9. Verfahren nach den Ansprüchen 1 bis 8, dadurch gekennzeichnet, daß die Isobuttersäure in Strömungsrichtung hinter der Einleitungsstelle des Sauerstoffs in das Wirbelbett eingeleitet wird.

10. Verfahren nach Anspruch 9, dadurch gekennzeichnet, daß Isobuttersäure und/oder Sauerstoff an mehreren, in Strömungsrichtung aufeinanderfolgenden Stellen eingeleitet werden.

11. Verfahren nach Anspruch 10, dadurch gekennzeichnet, daß ein Katalysator eingesetzt wird, der ein Trägermaterial mit einer spezifischen Oberfläche von weniger als 2 qm/g enthält.

12. Verfahren nach den Ansprüchen 1 bis 11, dadurch gekennzeichnet, daß die bei der Oxydehydrierung freigesetzte Reaktionswärme zum Teil im Bereich des Wirbelbettes und zum Teil im Gasraum oberhalb des Wirbelbettes entnommen wird.

13. Verfahren nach den Ansprüchen 1 bis 12, dadurch gekennzeichnet, daß ein Teil der aus dem Wirbelbett abströmenden Gasphase - gegebenenfalls nach Abtrennung eines Teils seiner Bestandteile - in das Wirbelbett zurückgeleitet wird.

14. Verfahren nach den Ansprüchen 1 bis 13, dadurch gekennzeichnet, daß das Reaktionsgas durch mehrere hintereinandergeschaltete Wirbelbetten geleitet wird.

15. Verfahren nach Anspruch 14, dadurch gekennzeichnet, daß dem Gasstrom zwischen zwei Wirbelbetten Sauerstoff und/oder Isobuttersäure-Dampf zugesetzt wird.

## Claims

1. A continuous process for dehydrating isobutyric acid to form methacrylic acid by reaction with oxygen in the gaseous phase on a heterogenous catalyst in the fluid bed, characterised in that a catalyst is used with a Mo-V-P-heteropolyacid as active substance and less than 2 mol of water vapour are used per mol of isobutyric acid.

2. A process according to claim 1, characterised in that fresh catalyst material is added during the continuous operation.

3. A process according to claim 2, characterised in that a part of the catalyst is continuously replaced.

4. A process according to claim 2, characterised in that a material obtained by regenerating an old catalyst is used as fresh catalyst material.

5. A process according to claim 4, characterised in that the catalyst removed from the fluid bed is treated with oxygen in a regeneration fluid bed and then returned to the first fluid bed.

6. A process according to claim 5, characterised in that the gaseous phase discharged from the regeneration fluid bed is introduced into the first fluid bed.

7. A process according to claims 1 to 6, characterised in that the reaction is carried out at below atmospheric pressure.

8. A process according to claim 7, characterised in that it is carried out at a maximum pressure of 700 mbar.

9. A process according to claims 1 to 8, characterised in that the isobutyric acid is introduced into the fluid bed, in the direction of flow, behind the point at which the oxygen is introduced.

10. A process according to claim 9, characterised in that isobutyric acid and/or oxygen is introduced at several points following one another in the direction of flow.

11. A process according to claim 10, characterised in that a catalyst is used which contains a carrier material with a specific surface of less than 2 qm/g.

12. A process according to claims 1 to 11, characterised in that the reaction heat released during oxydehydration is removed partly from the region of the fluid bed and partly from the gas space above the fluid bed.

13. A process according to claims 1 to 12, characterised in that a part of the gaseous phase discharged from the fluid bed is returned to the fluid bed, optionally after separating a part of its components.

14. A process according to claims 1 to 13, characterised in that the reaction gas is passed through several fluid beds connected in series.

15. A process according to claim 14, characterised in that oxygen and/or vapour of isobutyric acid is added to the gas current between two fluid beds.

## Revendications

1. Procédé continu de déshydrogénation d'acide isobutyrique en acide méthacrylique par réaction avec de l'oxygène en phase gazeuse, en présence d'un catalyseur hétérogène en lit fluidisé, caractérisé en ce qu'on utilise un catalyseur renfermant un hétéropolyacide de Mo-V-P comme substance active, et en ce qu'on utilise moins de 2 moles de vapeur d'eau par mole d'acide isobutyrique.

2. Procédé selon la revendication 1, caractérisé en ce que de la matière catalytique fraîche est ajoutée pendant l'opération menée en continu.

3. Procédé selon la revendication 2, caractérisé en ce qu'une partie du catalyseur est remplacée de façon continue.

4. Procédé selon la revendication 2, caractérisé en ce qu'on utilise, comme matière catalytique fraîche, une matière catalytique récupérée par régénération de catalyseur ayant déjà servi.

5. Procédé selon la revendication 4, caractérisé en ce que le catalyseur prélevé dans le lit fluidisé est traité par de l'oxygène dans un lit fluidisé de régénération, puis est renvoyé dans le premier lit fluidisé.

6. Procédé selon la revendication 5, caractérisé en ce que la phase gazeuse qui s'écoule du lit fluidisé de régénération est envoyée dans le premier lit fluidisé.

7. Procédé selon l'une quelconque des revendications 1 à 6, caractérisé en ce que la réaction est conduite sous une pression inférieure à la pression atmosphérique.

8. Procédé selon la revendication 7, caractérisé en ce qu'il est conduit sous une pression de 700 mbar au maximum.

9. Procédé selon l'une quelconque des revendications 1 à 8, caractérisé en ce que l'acide isobutyrique est introduit dans le lit fluidisé en aval du point d'introduction de l'oxygène dans le sens de l'écoulement.

10. Procédé selon la revendication 9, caractérisé en ce que de l'acide isobutyrique et/ou de l'oxygène sont introduits en plusieurs points successifs dans le sens de l'écoulement.

11. Procédé selon la revendication 10, caractérisé en ce qu'on utilise un catalyseur qui contient une matière de support ayant une surface spécifique de moins de 2 m²/g.

12. Procédé selon l'une quelconque des revendications 1 à 11, caractérisé en ce que la chaleur de réaction dégagée au cours de l'oxydéshydrogénation est prélevée en partie dans la région du lit fluidisé et en partie dans l'atmosphère au-dessus du lit fluidisé.

13. Procédé selon l'une quelconque des revendications 1 à 12, caractérisé en ce qu'une partie de la phase gazeuse qui s'écoule du lit fluidisé est renvoyée dans le lit fluidisé - éventuellement après séparation d'une partie de ses constituants.

14. Procédé selon l'une quelconque des revendications 1 à 13, caractérisé en ce que le gaz réactionnel est envoyé à travers plusieurs lits fluidisés disposés en série.

15. Procédé selon la revendication 14, caractérisé en ce que de l'oxygène et/ou de la vapeur d'acide isobutyrique sont ajoutés au courant de gaz entre deux lits fluidisés.
